# EUROPEAN PATENT SPECIFICATION

(11) **EP 0 832 096 B1**
(45) Date of publication and mention of the grant of the patent: **18.07.2001**
(21) Application number: 95919029.9
(22) Date of filing: 04.05.1995
(51) Int. Cl.: C07K 1/02, C07K 1/04, C07K 1/08, C07K 1/13, C07K 17/06, C08G 69/10, C08L 89/00

(54) **SYNTHESIS OF PROTEINS BY NATIVE CHEMICAL LIGATION**
PROTEINSYNTHESE MITTELS NATIVER CHEMISCHER LIGATION (07.01.97)
SYNTHESE DE PROTEINES PAR LIGATURE CHIMIQUE ENDOGENE

(43) Date of publication of application: 01.04.1998
(73) Proprietor: The Scripps Research Institute, La Jolla, CA 92037 (US)
(72) Inventor: KENT, Stephen, B., H., La Jolla, CA 92037 (US); MUIR, Tom, W., San Diego, CA 92106 (US); DAWSON, Philip, E., La Jolla, CA 92037 (US)
(74) Representative: Hedley, Nicholas James Matthew
(86) International application number: US9505668
(87) International publication number: WO9634878

(56) References cited:
- US-A- 4 910 222
- US-A- 5 399 501
- P E DAWSON ET AL.: "Synthesis of proteins by native chemical ligation" SCIENCE., vol. 266, 4 November 1994, LANCASTER, PA US, pages 776-779, XP002064666
- M BACA ET AL.: "Chemical ligation of cysteine-containing peptides; synthesis of a 22 kDa tethered dimer of HIV-1 protease " JOURNAL OF THE AMERICAN CHEMICAL SOCIETY., vol. 117, no. 7, 22 February 1995, DC US, pages 1881-1887, XP000611590
- J P TAM ET AL.: "peptide synthesis using unprotected peptides though orthogonal coupling methods" PROCEEDINGS OF THE NATIONAL ACADEMY OF SCIENCES OF USA., vol. 92, no. 26, 19 December 1995, WASHINGTON US, pages 12485-12489, XP002064667
- L E CANNE ET AL.: "Extending the applicability of native chemical ligation" JOURNAL OF THE AMERICAN CHEMICAL SOCIETY., vol. 118, no. 25, 26 June 1996, DC US, pages 5891-5896, XP002064668
- TETRAHEDRON LETTERS, Volume 36, Nummber 8, issued 20 February 1995, CANNE et al., "A General Method for the Synthesis of Thioester Resin Linkers for Use in the Solid Phase Synthesis of Peptide-alpha-Thioacids", pages 1217-1220.
- SCIENCE, Volume 256, issued 10 April 1992, SCHNOLZER et al., "Constructing Proteins by Dovetailing Unprotected Synthetic Peptides: Backbone-Engineered HIV Protease", pages 221-225.
- PROC. NATL. ACAD. SCI. U.S.A., Volume 83, issued March 1986, ABBOTT et al., "Intrahepatic Transport and Utilization of Biliary Glutathione and its Metabolites", pages 1246-1250.

## Description

### Field of Invention:

The invention relates to methods and intermediates for chemically ligating two oligopeptides end to end with an amide bond. More particularly, the invention relates to methods and intermediates for chemically ligating oligopeptides wherein an unoxidized N-terminal cysteine of a first oligopeptide condenses with a C-terminal thioester of a second oligopeptide to form a β-aminothioester intermediate which spontaneously rearranges intramolecularly to form an amide bond and the ligation product.

### Government Rights:

The invention disclosed herein was supported in part by Grants Number R01 GM 48897-01, Number P01 GM 48870-03, and Number GM 50969-01 from the National Institutes of Health. The United States government may have certain rights to this invention.

### Background:

Proteins may be synthesized chemically, ribosomally in a cell free system, or ribosomally within a cell. Advances in each of these areas have significantly improved access to many proteins but have also stimulated demand for yet further improvements.

Proteins owe their diverse properties to the precisely folded three dimensional structures of their polypeptide chains. The three dimensional structure of a protein determines its functional attributes. However, at present, it is difficult to predict and/or fully explain the biological properties of a protein from its three dimensional structure alone. A better understanding of how structure determines the biological properties of a protein can be achieved by systematically varying the covalent structure of the molecule and correlating the effects with the folded structure and biological function. Accordingly, there is an increased demand for enhanced synthetic techniques for synthesizing new proteins and protein analogs.

Techniques derived from recombinant DNA-based molecular biology can be employed to facilitate the expression of proteins in genetically engineered micro-organisms. The use of site-directed mutagenesis, as disclosed by M. Smith (*Angew. Chem. Int. Ed. Engl.* (1994): vol. 33, p 1214), enables the preparation of large numbers of modified proteins in useful amounts for systematic study, e.g., C. Eigenbrot and A. Kossiakoff, *Current Opinion in Biotechnology* (1992): vol. 3, p 333. The use of innovative approaches increases the range of amino acids that can be incorporated in expression systems and promises to significantly extend the utility of biosynthetic modification of the covalent structure of proteins. (C. J. Noren et al., *Science* (1989): vol. 244, p 182 (1989); J. A. Ellman et al., *Science* (1992): vol. 255, p 197.) However, there appear to be limitations inherent to the nature of ribosomal protein synthesis. (V. W. Cornish, et al., *Proc. Natl. Acad. Sci. USA* (1994): vol. 91, p 2910.)

Chemical synthesis of proteins has also contributed to the exploration of the relationship of protein structure to function. Stepwise solid phase synthesis has permitted the *de novo* preparation of small proteins. (T. W. Muir et al., *Curr. Opin. Biotech.* (1993): vol. 4, p 420.) There are also several examples of the use of stepwise solid phase synthesis of whole proteins to explore the molecular basis of biological function. (M. Miller, et al., *Science* (1989): vol. 246, p 1149; A. Wlodawer, et al., *Science* (1989): vol. 245, p 616; L.H. Huang, et al., *Biochemistry* (1991): vol. 30, p 7402; and K. Rajarathnam, et al., *Science* (1994): vol. 264, p 90.)

Semi-synthesis through the conformationally-assisted religation of peptide fragments can also be employed, in special instances, to study of the structure/function relationship of proteins. (R. E. Offord, "Chemical Approaches to Protein Engineering", in Protein Design and the Development of New therapeutics and Vaccines, J. B. Hook, G. Poste, Eds., (Plenum Press, New York, 1990) pp. 253-282; C. J. A. Wallace, et al., *J. Biol. Chem.* (1992): vol. 267, p 3852. An important extension of the semisynthesis approach is the use of enzymatic ligation of cloned or synthetic peptide segments. (L. Abrahmsen, et al., *Biochemistry* (1991): vol. 30, p 4151; T. K. Chang, et al., *Proc. Natl. Acad. Sci. USA* (1994): in press.) Although the above methodologies have been successfully applied to the synthesis of proteins and protein analogs, T. W. Muir et al., report that there is a continued interest in the wider application of the tools of organic chemistry to the study of proteins (*Curr. Opin. Biotech.* (1993): vol. 4, p 420.)

Stephen Kent et al. recently introduced the chemical ligation of unprotected peptide segments as an improved route to the total synthesis of proteins. (M. Schnlzer, et al., *Science* (1992): vol., 3256, p 221.) Chemical ligation involves the chemoselective reaction of unprotected peptides to give a product with an unnatural backbone structure at the ligation site. Use of unprotected peptides circumvented the difficulties inherent to classical chemical synthesis, viz complex combinations of protecting groups that lead to limited solubility of many synthetic intermediates, e.g. K. Akaji, et al., *Chem. Pharm. Bull. (Tokyo)* (1985): vol. 33, p 184. In contrast, the chemical ligation technique has allowed us to make good use of the ability to routinely make, purify, and characterize unprotected peptides 50 or more residues in length. Using optimized stepwise solid phase methods the preparation in good yield and high purity of peptides up to 60 residues is routine. In favorable cases, peptides of 80+ residues can be prepared. (M. Schnolzer, et al., *Int. J. Pept. Prot. Res.* (1992): vol. 40, p 180-193.)

The key aspect of the above approach to chemical ligation is the use of a chemoselective reaction to specifically and unambiguously join peptides by formation of an unnatural (i.e. non-peptide) backbone structure at the ligation site. It has permitted the facile preparation of a wide range of backbone-modified proteins, including analogues of protein domains, e.g., ligated 10F3, the integrin-binding module of fibronectin: 95 residues (M. Williams, et al., *J. Am. Chem. Soc.* (1994): in press.) The catalytic contribution of flap-substrate hydrogen bonds in HIV-1 protease has been elucidated by the chemical synthesis of a homodimer of 99 residue subunits of this protein by chemical ligation. (M. Baca, et al., *Proc. Natl. Acad. Sci. U.S.,* (1993): vol. 90, p 11638.) Chemical ligation has also proven to be useful for the routine, reproducible synthesis of large amounts of proteins in high purity with full biological activity (20). (R. C. deLisle Milton, et al., "Synthesis of Proteins by Chemical Ligation of Unprotected Peptide Segments: Mirror-Image Enzyme Molecules, D- & L-HIV Protease Analogs," in Techniques in Protein Chemistry IV, Academic Press, New York, pp. 257-267 (1992).)

Chemical ligation can also be employed for the straightforward production of protein-like molecules of unusual topology, e.g., four-helix bundle template-assembled synthetic protein (MW 6647 Da) (P. E. Dawson, et al., *J. Am. Chem. Soc.* (1993): vol. 115, p 7263); homogeneous multivalent artificial protein (MW 19,916 Da) (K. Rose, *J. Am. Chem. Soc.* (1994): vol. 3116, p 30); artificial neoprotein mimic of the cytoplasmic domains of a multichain integrin receptor (MW 14,194 Da) (T. W. Muir, et al., *Biochemistry,* (1994): vol. 33, pp 7701-7708; and peptide dendrimer (MW 24,205 Da) (C. Rao, et al., *J. Am. Chem. Soc.* (1994): vol. 116, p 6975. The range of proteins accessible by this technique is limited by the size of the synthetic peptide segments.

A useful extension would occur if one had direct synthetic access to native backbone polypeptide chains up to the size of typical protein domains. (A. L. Berman, et al., *Proc. Natl. Acad. Sci. USA* (1994): vol. 91, p 4044.) Chemical ligation would then be employed to string these domains together to explore the world of proteins in a general fashion.

A modular strategy for the total synthesis of proteins has been developed, based on the convergent chemical ligation of unprotected peptides has been disclosed by L.E. Canne, et al. (presented at the Annual Meeting of the Protein Society, San Diego, July 1994). Protein domains (modules) were prepared by chemical ligation of 50-70 residue segments; these domains were then stitched together to give the target protein. Mutually compatible ligation chemistries are required: intra-domain ligation should optimally yield a stable, peptide-like bond; inter-domain ligation will tolerate a wider variation of properties of the structure formed at the ligation site. Dawson et al., Science 266, pages 776-779, discuss a sampler method of native chemical ligation.

Straightforward total chemical synthesis of proteins represents the realization of an important objective of organic chemistry. It raises the exciting prospect of unrestricted variation of protein covalent structure made possible by general synthetic access, and will give new impetus to exploration of the structural basis of properties such as folding, stability, catalytic activity, binding, and biological action.

What is needed is a technique of native chemical ligation which combines the formation of a native peptide bond at the ligation site with the advantages of chemoselective reaction of unprotected peptides. This second generation ligation chemistry would significantly increase the size of native backbone polypeptides directly accessible by total chemical synthesis. It could be usefully applied to a wide range of synthetic targets, including proteins of moderate size, and it allows direct access to protein functional domains. Native chemical ligation is a foundation stone of a general modular approach to the total chemical synthesis of proteins. Furthermore, it is compatible with the use of both chemically synthesized peptides and peptide segments derived from other sources.

### Summary:

One aspect of the invention is directed to a method of native chemical ligation. The method of native chemical ligation facilitates the chemical synthesis of proteins and large oligopeptide. The principle of 'native chemical ligation' is shown in Scheme 1. The first step is the chemoselective reaction of an unprotected synthetic peptide-α-thioester with another unprotected peptide segment containing an N-terminal Cys residue, to give a thioester-linked intermediate as the initial covalent product. Without change in the reaction conditions, this intermediate undergoes spontaneous, rapid intramolecular reaction to form a native peptide bond at the ligation site. The target full length polypeptide product is obtained in the desired final form without further manipulation. The general synthetic access provided by the method of native chemical ligation greatly expands the scope of variation of the covalent structure of the protein molecule.

The invention provides a method for ligating a first oligopeptide with a second oligopeptide end to end for producing an oligopeptide product. The first and second oligopeptides are admixed in a reaction solution including a catalytic thiol. The catalytic thiol is a conjugated thiol attached directly to an aromatic or heteroaromatic ring and is different from the thiol from which the C-terminal thioester is nationally formed. Preferred catalytic thiols include thiophenol, 1-thio-2-nitrophenol, 2-thio-benzoic acid, 2-thio-pyridine, 4-thio-2-pyridinecarboxylic acid, and 4-thio-2-nitro-pyridine. The first oligopeptide includes a C-terminal thioester. The second oligopeptide includes an N-terminal cysteine having an unoxidized sulfhydryl side chain. The unoxidized sulfhydryl side chain of the N-terminal cysteine is then condensed with the C-terminal thioester to produce an intermediate oligopeptide which links the 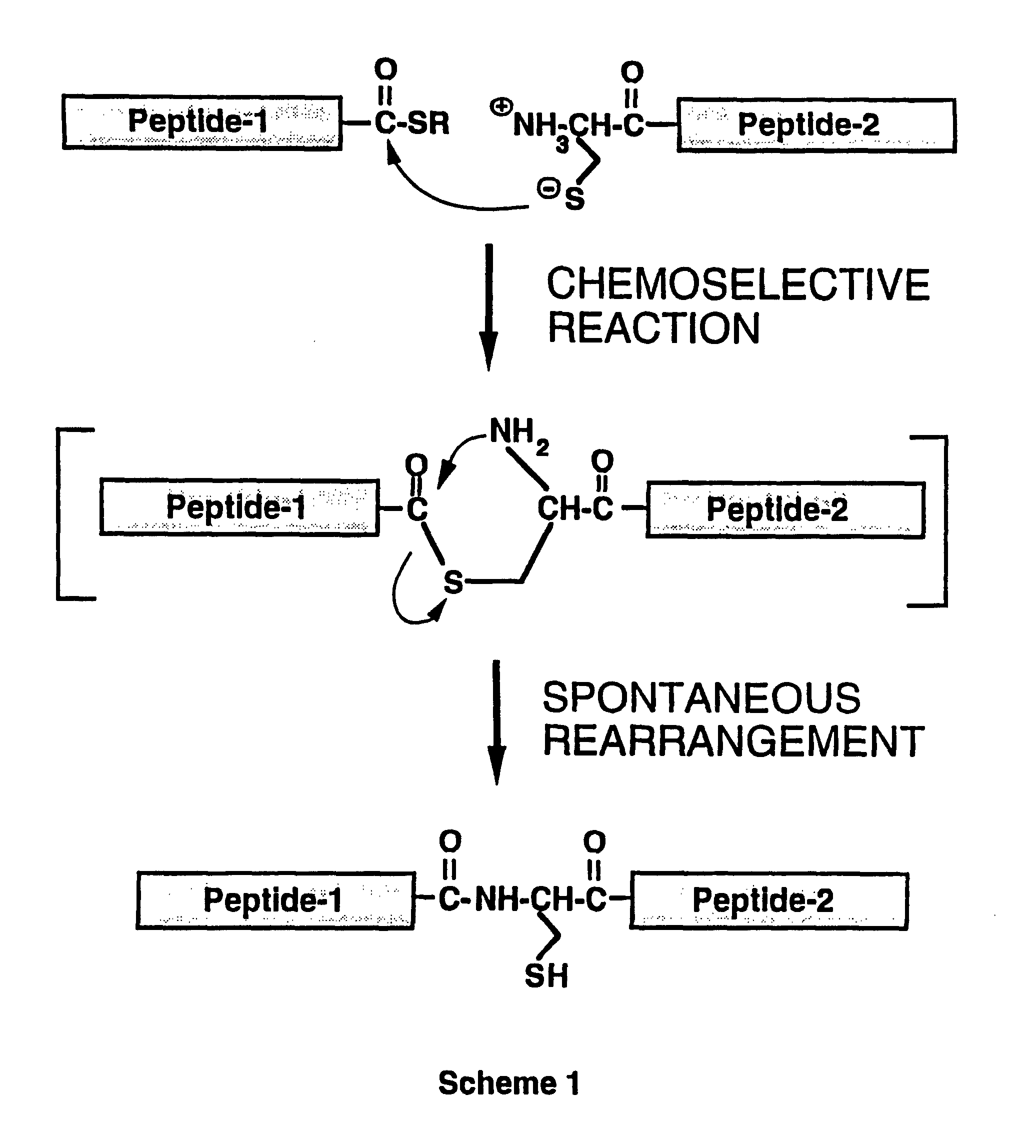 first and second oligopeptides with a β-aminothioester bond. The β-aminothioester bond of the intermediate oligopeptide then undergoes an intramolecular rearrangement to produce the oligopeptide product which links the first and second oligopeptides with an amide bond.

The oilgopeptide having a C-terminal thioester referred to above is prepared according to a method which admixes a resin having a linker with an unoxidized thiol with a Boc-amino acid succinimide ester under reaction conditions to produce a Boc-amino thioester-resin. An oligopeptide is then assembled onto the Boc-amino thioester-resin by stepwise solid phase peptide synthesis. When the oligopeptide is complete, the the Boc-amino thioester-resin is cleaved with HS to produce an oligopeptide having a C-terminal thiol. The C-terminal thiol is then converted to an oligopeptide having a C-terminal thioester.

The oligopeptide thioester (α-COSR moiety) or Scheme 1 can be readily generated from a corresponding oligopeptide thiol (-αCOSH) prepared by highly optimized stepwise SPPS on a thioester resin. The thioester resin was prepared by the method of L.E. Canne et al., *Tetrahedron Letters* (1995): vol. 36, pp. 1217-1220, incorporated herein by reference. The method of Canne employs the thioester resin disclosed by Blake and Yamashiro (J. Blake, *Int. J. Pept. Protein Res.* (1981): vol. 17, p 273; D. Yamashiro, et al., *Int. J. Pept. Protein Res.* (1988): vol. 31, p 322). Peptide products were cleaved, purified, and characterized by conventional methods. (M. Schnolzer, et al., *Int. J. Pept. Protein* Res.,(1992): vol. 40, pp 180-193.)

The Yamashiro methodology activates a thiocarboxyl group on a protected oligopeptide with diaryl disulfides to give acyl disulfides (Yamashiro et. al. *Int. J. Peptide Protein Res.* (1922): vol. 31, pp 322-334). These C-terminal-peptide-acyl-disulfides are highly reactive electrophilic intermediates which are attacked and subsequently coupled with an α-amino group on the N-terminus of a second peptide to form native peptide bonds. The reported coupling yields using 2,2'-dipyridyl disulfide as the activator of the thiocarboxyl group afford the desired α-IB-92 product in 45% yield. Overall yields based on the starting resin for a 3-segment synthesis of α-IB-92 are reported as 8%, while a 2-segment synthesis gave 11%.

Due to the high reactivity of the diaryl disulfide bond, the Yamashiro approach requires extensive protection and deprotection of amino acid residues present in the peptide molecule. The lysine group for example is protected as a citraconyl derivative because of the reactive amine functionality. Additionally, an Msc group or tBOC group is used to protect any terminal amine functionalities present in the molecule.

The invention stated herein does not require the use of any protecting groups for the coupling of two oligopeptides because a less reactive (and thus more chemoselective) thioester electrophile is used instead of the acyl disulfide moiety (Yamashiro's approach). In the intermolecular coupling step, this thioester electrophile requires a more nucleophilic sulfhydryl moiety rather than a free amine. The nucleophilic sulfhydryl moiety can be found on cysteine residues. Since the amino and hydroxyl functionalities are relatively unreactive to the thioester electrophile, a selective coupling of the two unprotected oligopeptides is achieved with the cysteine sulfhydryl moiety. The sulfhydryl group on the cysteine of peptide 2 will first attack the thioester of peptide 1 and form a coupled thioester intermediate. This coupled thioester intermediate is concomitantly attacked by the free α-amino moiety from the cysteine and spontaneously rearranges to form the native peptide bond. Yields are therefore increased by eliminating protection and deprotection steps, since side undesired reactions are reduced (Scheme 1).

The thioester moiety is prepared from a precursor thioacid which is obtained by optimized stepwise solid-phase peptide synthesis on a aminomethyl resin support equipped with a thioester resin linker. The precursor thioacid is subsequently generated in liquid HF at 0 °C in 1 hour (Yamashiro et. al. *Int. J. Pept. Protein Res.* (1988): vol. 31, p 322).

A procedure for the synthesis of the thioester linker with use of a stepwise solid phase peptide synthesis has been reported by Blake (Blake et. al. *Proc. Natl. Acad. Sci. USA* (1981): vol. 78, 4055) and Yamashiro (Yamashiro et. al. *Int. J. Pept. Protein Res.* (1988): vol. 31, p 322). This method is undesirable, however, because it requires the conversion of Boc-amino acid succinimide esters to the corresponding Boc-amino thioacids with hydrogen sulfide. An improved methodology reported herein, utilizes the Boc-amino acid succinimide ester directly and therefore avoids the inconvenience and hazards of hydrogen sulfide gas (Kent et. al. *Tetrahedron Lett.* (1995): vol. 36, p 1217).

In this method (**Scheme 2**), thiol **3** is generated from the reaction of chloride **2** (Yamashiro et. al. *Int. J. Pept. Protein Res.* (1988) : vol. 31, p 322) with thiourea, followed by hydrolysis of the resulting thiouronium salt in aqueous base. Thiol **3** is a general intermediate which can be reacted with a wide range of commercially available Boc-amino acid succinimide esters to produce the desired thioester linker **1** which is conveniently isolated as the dicyclohexylamine (DCHA) salt.

Model studies were undertaken with small peptides to investigate the native chemical ligation approach. To help explore the mechanism of the reaction, the peptide Leu-Tyr-Arg-Ala-Gly-α-COSBzl was reacted with Ac-Cys. The exact mass of the resulting ligation product was determined by electrospray mass spectrometry, and was consistent with a thioester-linked peptide as the ligation product generated by nucleophilic attack of the Ac-Cys side chain on the a-thioester moiety of the peptide. Reaction of Leu-Tyr-Arg-Ala-Gly-α-COSBzl with H-Cys-Arg-Ala-Glu-Tyr-Ser (containing an unblocked a-NH2 functional group) proceeded rapidly at pH 6.8 (below pH 6 the reaction proceeded very slowly, suggesting the involvement of the ionized thiolate form of the Cys side chain), and gave a single product of the expected mass. This product lacked susceptibility to nucleophiles, and had the ability to form disulfide-linked dimeric peptides, indicating unambiguously the formation of a native amide bond at the ligation site. These studies were consistent with the mechanism shown in Scheme 1, in which the initial thioester ligation product was not observed as a discrete intermediate because of the rapid rearrangement to form a stable peptide bond. Facile intramolecular reaction results from the favorable geometric arrangement of the α-NH₂ moiety with respect to the thioester formed in the initial chemoselective 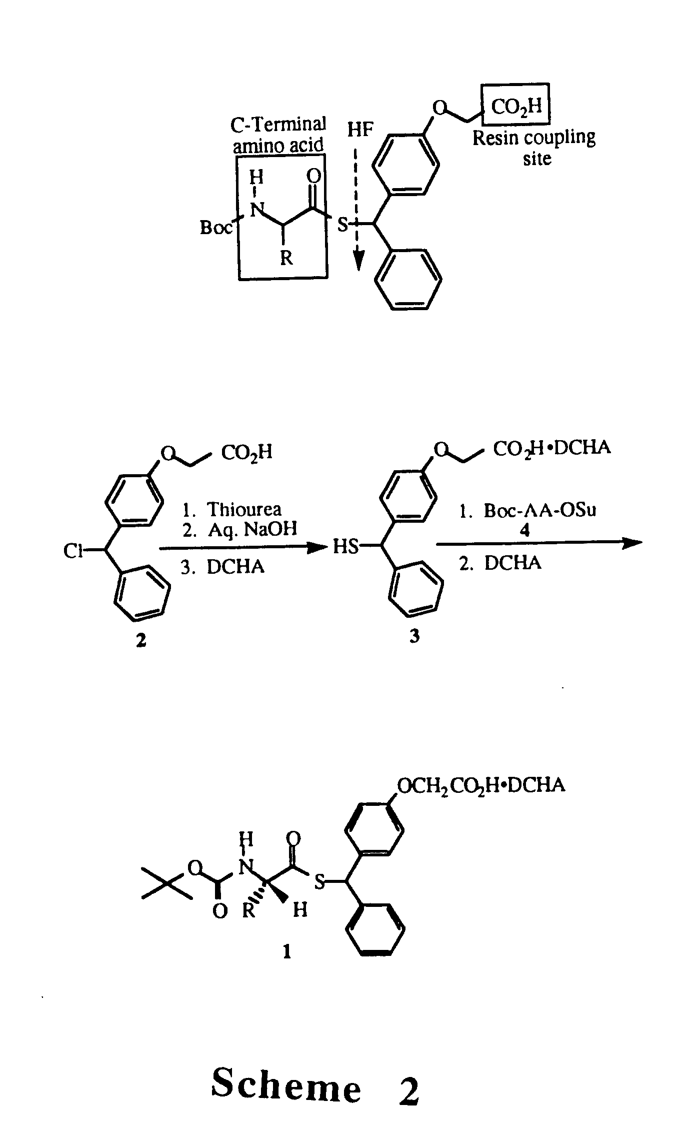 ligation reaction. Use of such 'entropy activation' for peptide bond formation is based on principles enunciated by Brenner. (M. Brenner, in Peptides. Proceedings of the Eighth European Peptide Symposium H. C. Beyerman, Eds. (North Holland, Amsterdam, 1967) pp. 1-7.) The concept of 'entropy activation' for peptide bond formation has been more recently adopted by D. S. Kemp et al. (*J. Org. Chem*. (1993): vol. 58, p 2216) and by C.-F. Liu, et al. (*J. Am. Chem. Soc.* (1994): vol. 116, p 4149).

Several model peptides have been synthesized by the method of native chemical ligation. The successful synthesis of these model peptides establish that native chemical ligation is generally applicable to peptides containing the full range of functional groups normally found in proteins. Even free internal Cys residues may be present in either of the reacting segments. Internal Cys residues can undergo ester exchange with the peptide-a-thioester component; however, this reaction is unproductive because no rearrangement to the amide bond can occur; the thioester formed is readily reversible and remains a productive part of the reacting system. As disclosed herein, native chemical ligation is limited to reaction at an N-terminal Cys residue. It is important to prevent the side chain thiol of this Cys from oxidizing to form a disulfide linked dimer, because this is unreactive in the ligation. An excess of thiol corresponding to the thioester leaving group was used to keep the Cys residues in reduced form without interfering with the ligation reaction. The amino-terminal peptide segment must be prepared by chemical synthesis to equip it with the necessary α-COSR functionality. Furthermore, for optimal ligation, this component should have an unhindered (i.e. non β-branched) C-terminal amino acid. Solubilizing agents such as urea or guanidine hydrochloride did not interfere with the ligation and 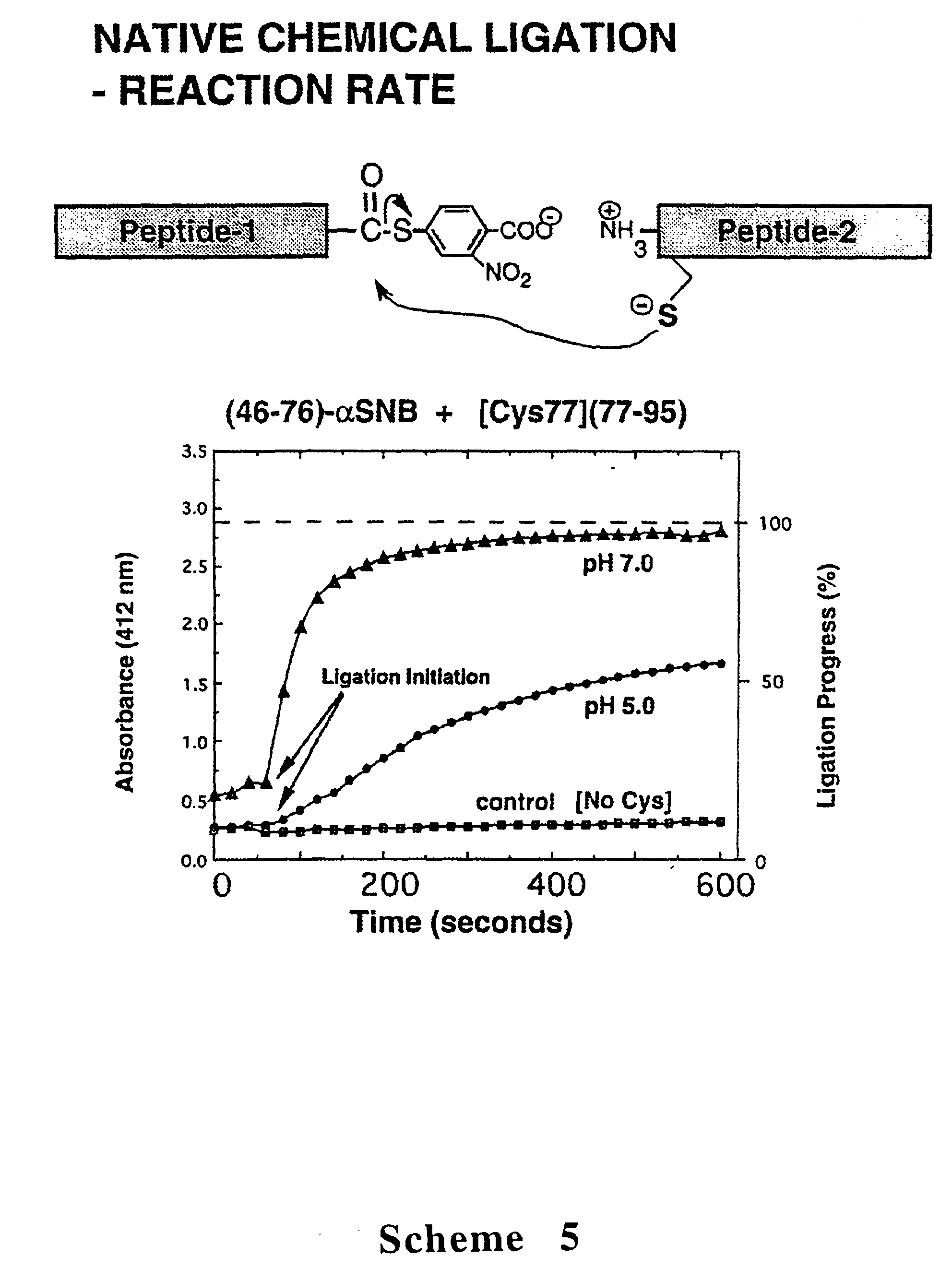 could be used to enhance the concentration of peptide segments, and thus increase the reaction rate.

Further model reactions demonstrate that the use of better thioester leaving groups results in faster ligation reactions. We applied this observation to the native chemical ligation of peptides from the extracellular domain of a human cytokine receptor (R. D'Andrea, et al., *Blood,* (1994): vol. 83, p 2802.) as shown in Scheme 5. Use of the 5-thio-2-nitrobenzoic acid (-SNB) leaving group, corresponding to the reduced form of Elman's reagent, gave rapid high yield reaction. As described below in connection with Scheme 5, the reaction between the peptide segments was observed to have gone essentially to completion in less than 5 minutes, giving the 50 residue product with a native peptide bond at the site of ligation. Thus, rapid native chemical ligation can be achieved by use of a thioester leaving group with suitably tuned properties.

Application of the native chemical ligation method to the total synthesis of a protein molecule was illustrated by the preparation of human interleukin 8 (IL-8). (M. Baggiolini, et al., *FEBS Lett.* (1989): vol. 307, p 97; I. Clark-Lewis, et al., *J. Biol. Chem.* (1994): vol. 269, p 16075 (1994); I. Clark-Lewis, *Biochemistry* (1991): vol. 30, p 3128; and K. Rajarathnam, et al., (1994): *Biochemistry,* (1994): vol. 29, p 1689.) The 72 amino acid polypeptide chain contains four Cys residues, which form two functionally critical disulfide bridges in the native protein molecule. The total synthesis of IL-8 is shown in Scheme 7. The two unprotected synthetic peptide segments reacted cleanly to give the full length polypeptide chain in reduced form without further chemical manipulation (9). This successful ligation was particularly significant because the 33- and 39-residue IL-8 segments each contained two Cys residues, and 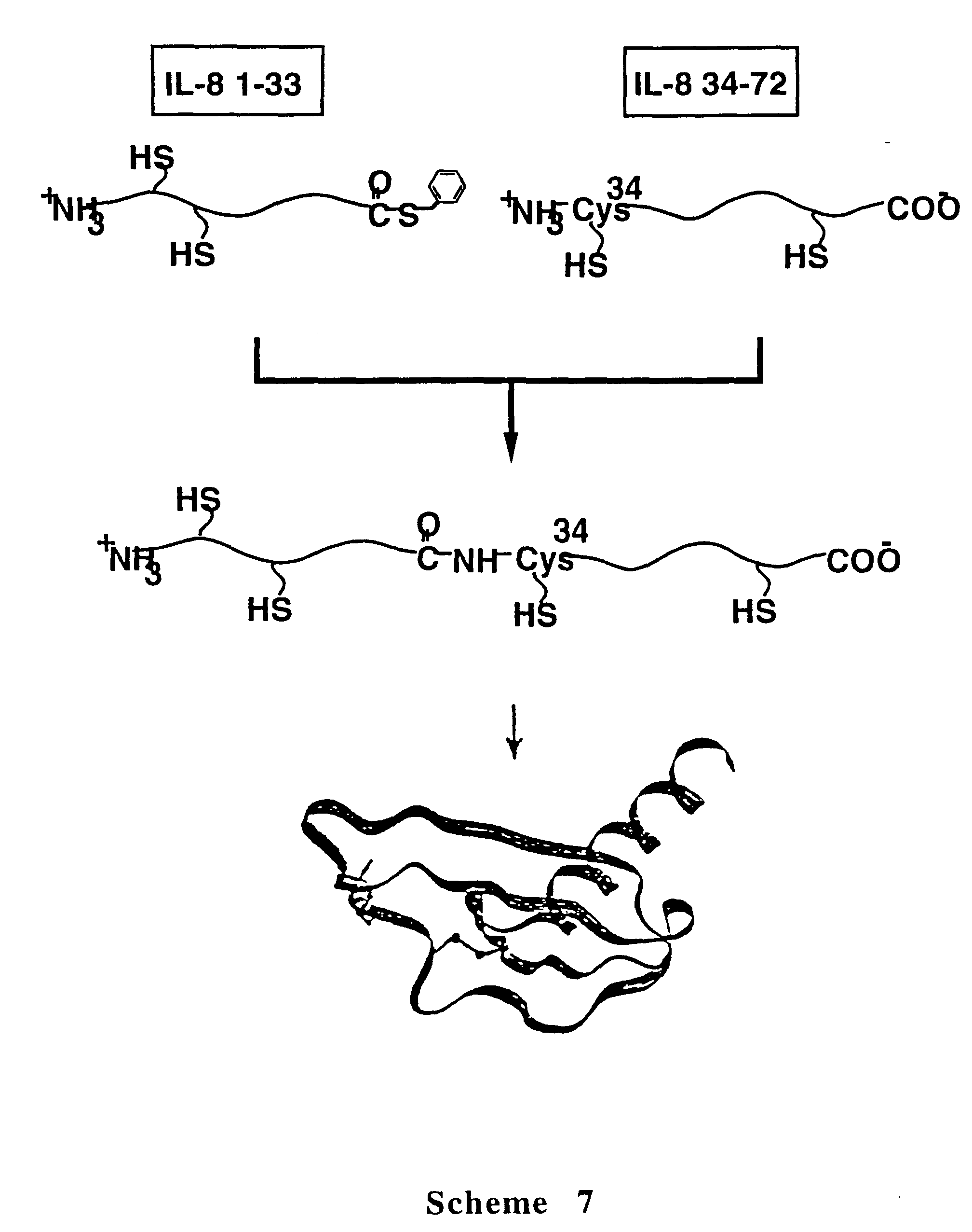 together encompassed 18 of the 20 genetically encoded amino acids found in proteins. The purified product was folded and oxidized as previously described, to give IL-8 with a mass precisely 4 daltons less than that of the original ligation product, indicating the formation of two disulfide bonds. The properties of this folded product were identical to those of previously studied authentic IL-8 samples. Titration in an assay for neutrophil elastase release demonstrated that the potencies (ED50 = 0.3nM) and maximal responses of the folded, ligated [Ala33]IL-8 and the corresponding molecule obtained by conventional synthesis were indistinguishable and identical to native sequence IL-8. This result unambiguously confirmed the formation of a peptide bond at the ligation site, because the thioester-to-amide rearrangement must have taken place to give the free Cys³⁴ side chain that formed the native disulfide bond (see Scheme 7).

Proteins are usually studied by expression in genetically engineered micro-organisms using the methods of recombinant DNA-based molecular biology. Methods such as site-directed mutagenesis have had a major impact on the ability to prepare large numbers of modified proteins in useful amounts for systematic study. Innovative approaches have increased the range of amino acids that can be incorporated in expression systems and promise to significantly extend the utility of biosynthetic modification of the covalent structure of proteins. However, there appear to be limitations inherent to the nature of ribosomal protein synthesis.

Wieland discloses a method for synthesizing dipeptides using a thioester intermediate. (Wieland et. al. *Liebigs Ann. Chem.* (1953): vol. 580, p 159.) Wieland utilizes the reaction of *S*-glycyl-(or other un-branched aminoacyl-) thiophenols with cysteine. Thus, the sulfhydryl group on the cysteine residue first attacks the thioester of the *S*-glycyl-thiophenol and forms a coupled thioester intermediate. This coupled thioester intermediate is concomitantly attacked by the free α-amino moiety from the cysteine and spontaneously rearranges to form the native peptide bond.

A limitation of the Wieland approach is the size of the molecules utilized (only mono-amino acids are coupled to cysteine) and stems from the methodology used for the synthesis of the thioester. To form the thioester, Wieland's approach requires the activation of the terminal carboxylic acid as a mixed anhydride, acid chloride or thioacid. A problem arises if an acidic moiety is present in an amino acid residue such as Asp(D) and Glu(E). In these cases, the Wieland produces an undesired side reaction and therefore requires a complex protecting group strategy, particularly if oligopeptides are synthesized.

The invention described herein eliminates the need for an elaborate protecting group strategy since the oligopeptide-thioester moiety is derived from a precursor thioacid. This precursor thioacid (peptide-α-COSH) is synthesized by a standard stepwise solid-phase peptide synthesis on an aminomethyl resin support, equipped with a thioester resin linker. The precursor thioacid is cleaved from the linker/resin almost quantitatively (99%) in liquid HF at 0 °C for 1 hour.

The thioester peptide (peptide-α-COSR) can be synthesized in two general ways:
(1) Reaction of a crude lyophilized thioacid peptide (peptide-α-COSH) with Ellman's reagent (5,5'-dithiobis-2-nitrobenzoic acid, available from Aldrich company) at pH 5.5 (2.0 equivalents), 6M Guanidine in 100mM Na acetate buffer. This gives the SNB-thioester peptide (peptide-α-COSNB) which is subsequently purified by reversed phase high performance liquid chromatography (RPHPLC).
(2) Reaction of a crude lyophilized thioacid peptide (peptide-α-COSH) with benzyl bromide at pH 4.0, 6M guanidine and 100mM Na acetate buffer. The benzyl thioester (peptide-α-COSBn) is then purified by RPHPLC.

The conditions stated above, permit the formation of an unprotected oligonucleotide which is equipped with the activated thioester. Subsequent reaction with a second peptide containing a terminal cysteine residue, permits a facile coupling with the formation of a native peptide bond and can generate oligopeptide chains of 100 or more amino acid residues (Scheme 1).

In favorable cases, chemical synthesis has already made important contributions to the exploration of the relationship of protein structure to function. Stepwise solid phase synthesis has permitted the *de novo* preparation of small proteins (14) and there have been several notable examples of the use of this method of total protein synthesis to explore the molecular basis of biological function. Another method that has in special instances allowed chemistry to be applied to the study of proteins is semi-synthesis through the conformationally-assisted religation of peptide fragments. An important extension of the semisynthesis approach is the use of enzymatic ligation of cloned or synthetic peptide segments. Although these methods currently have severe limitations, there continues to be serious interest in the wider application of the tools of organic chemistry to the study of proteins.

Native chemical ligation provides precisely that capability. It combines the formation of a native peptide bond at the ligation site with the advantages of chemoselective reaction of unprotected peptides. This second generation ligation chemistry dramatically increases the size of native backbone polypeptides directly accessible by total chemical synthesis. It can be usefully applied to a wide range of synthetic targets, including proteins of moderate size, and it allows direct access to protein functional domains. Native chemical ligation is a foundation stone of a general modular approach to the total chemical synthesis of proteins. Furthermore, it is compatible with the use of both chemically synthesized peptides and peptide segments derived from other sources.

Straightforward total chemical synthesis of proteins represents the realization of an important objective of organic chemistry. It provides for unrestricted variation of protein covalent structure made possible by general synthetic access, and provides new impetus to exploration of the structural basis of properties such as folding, stability, catalytic activity, binding, and biological action.

In an alternative embodiment, the carboxy-terminal peptide segment or protein module can be expressed by standard recDNA means; provided the product contained an N-terminal Cys residue, it could be reacted with the synthetic amino-terminal peptide-α-COSR using the native chemical ligation described here to give a product in which part of the protein had derived from chemical synthesis and part from ribosomal synthesis.

### Detailed Description:

### Peptide-α-thioacid formation

A typical procedure for the formation and utilization of the thioester resin linker for use in the solid phase synthesis of peptide-α-thioacids is as follows: (Kent et. al. *Tetrahedron Lett.* (1995): vol. 36, p 1217).

**4-(α-Mercaptobenzyl)phenoxyacetic acid, dicyclohexylamine (3) Scheme 2.** A mixture of **2**, formed using the conditions as established by Yamashiro et. al. *Int. J. Pept. Protein Res.* (1988): vol. 31, pp 322-334, (7.5 grams, 27 mmol), thiourea (2.3 g, 30 mmol), and ethanol (100 mL) were heated to reflux (conditions as reported by Koenig et al *J. Org. Chem.* (1958): vol. 23, pp 1525-1530). After 4 hours, conversion to the thiouronium salt was essentially complete as shown by TLC (90:5:5 chloroform:Methanol:Acetic acid). 10N NaOH (30 ml) was added and the reflux continued for 2-3 hours. After cooling to room temperature, the reaction mixture was concentrated *in vacuo* to approximately half the original volume, acidified with concentrated HCl (to pH 2.0), and extracted with ethylacetate (4 x 30 mL). The combined ethylacetate extracts were washed with saturated NaCl (1 x 30 mL) and dried over MgSO₄. The volatile materials were removed *in vacuo.* The resulting oil was dissolved in ethylacetate (100 mL) and any insoluble material filtered. DCHA (dicyclohexylamine - available from Aldrich company), (6.0 mL, 30 mmol) was added to the filtrate with stirring. Within a few minutes, a white solid began to precipitate. Diethylether (150 mL) was added and the suspension cooled at -20 °C for several hours. The resulting white solid was filtered, washed with Diethylether, and dried under vacuum to give 3 (10.3 g, 23 mmol, 84%): ¹H NMR (CDCl₃): δ 7.30 (m, 7H), 6.82 (d, 2H, J=8.7 Hz), 5.39 (br s, 1H), 4.40 (s, 2H), 2.81 (m, 2H), 2.23 (br s, 1H, ex D₂O), 1.88-1.02 (comp m, 20H); FAB MS (cesium ion): calc for [C₂₇H₃₇NO₃S, H⁺] 456.2572, found 456.2572. Anal. Calcd for C₂₇H₃₇NO₃S: C, 71.17; H, 8.18; N, 3.07; S, 7.04. Found: C, 71.11; H, 8.41; N, 3.08; S, 7.09.

**General Synthesis of Boc-amino thioester linker (1), dicyclohexylamine salt (scheme 2).** A mixture of **3** (3.67 mmol), Boc-Ala-OSu (available from Novabiochem Corp.) (3.68 mmol), DIEA (diisopropylethylamine-5.74 mmol), dimethylformamide (35 mL) and methylene chloride (4 mL) was stirred at room temperature. After several hours, the initial white suspension completely dissolved to give a clear, colorless solution. After 24 hours, the reaction mixture was poured into 1N HCl (150 mL) and extracted with ethylacetate (4 x 35 mL). The combined ethylacetate extracts were washed with 1N HCl (2 x 30 mL), H₂O (1 x 30 mL), saturated NaCl (1 x 30 mL) and dried over MgSO₄. Volatiles were removed *in vacuo*. The resulting oil was purified by flash chromatography (925:50:25 Chloroform:MeOH:acetic acid) to give an oil contaminated with Acetic acid. To remove residual Acetic acid, the oil was dissolved in Chloroform (40 mL) and washed with 0.1 N HCl (7 x 10 mL), saturated NaCl (1 x 10 mL) and dried over MgSO₄. Volatiles were removed in *vacuo* to give **1** as an oil. This oil was dissolved in diethylether (10 mL) to which was added dicyclohexylamine (1 equivalents). Hexane (100 mL) was added with stirring to separate the dicyclohexylamine salt of **1** as a thick oil from any unreacted dicyclohexylamine. Solvents were decanted from the oil and the oil dissolved in CH₂Cl₂ (30-40 mL). The resulting solution was concentrated *in vacuo* to give the dicyclohexylamine salt **1** as a white foamy solid (scheme 2).

An example of the linkage and synthesis on the resin is as follows: 4-[a-(Boc-Ala-S)benzyl]phenoxyacetic acid (0.80 mmol) is added in 9 ml methylene chloride to 1.00 g aminomethyl-resin (0.40 mmol) and treated at 0 °C with 1.33 mL 0.6 M DCCI (dicyclohexylcarbodiimide) in methylene chloride for 15 min and at 24 °C for 30 minutes. The product is then subjected to standard solid-phase peptide synthesis conditions (Kent et. al. *Tetrahedron Letters* (1995): vol. 36, p 1217; J. Blake, *Int. J. Pept. Protein Res.* (1981): vol. 17, p 273). Once the desired chain is synthesized, the peptide resin (approx. 45 *µ*mol original load) is treated in 8 mL liquid HF (0.8 mL anisole) at 0 °C for 1 hour. After evaporation with nitrogen, the residue is washed with ethyl acetate. The solid is subsequently stirred in water (approx. 15 mL) at 0 °C while adjusting the pH to 6.0 with solid ammonium bicarbonate. Filtration and lyophilization gives the crude thioacid product which can be further purified by preparative HPLC in 30 mg batches.

### Preparation of the thioester terminal peptide segment

The α-COSR thioester peptide can be synthesized in two general ways:
(1) Reaction of a crude lyophilized thioacid peptide with Ellman's reagent (5,5'-dithiobis-2-nitrobenzoic acid, available from Aldrich company) at pH 5.5 (2.0 equivalents), 6M Guanidine in 100mM Na acetate buffer. This gives the SNB-thioester peptide which is subsequently purified by reversed phase high performance liquid chromatography (RPHPLC).
(2) Reaction of a crude lyophilized thioacid peptide with benzyl bromide at pH 4.0, 6M guanidine and 100mM Na 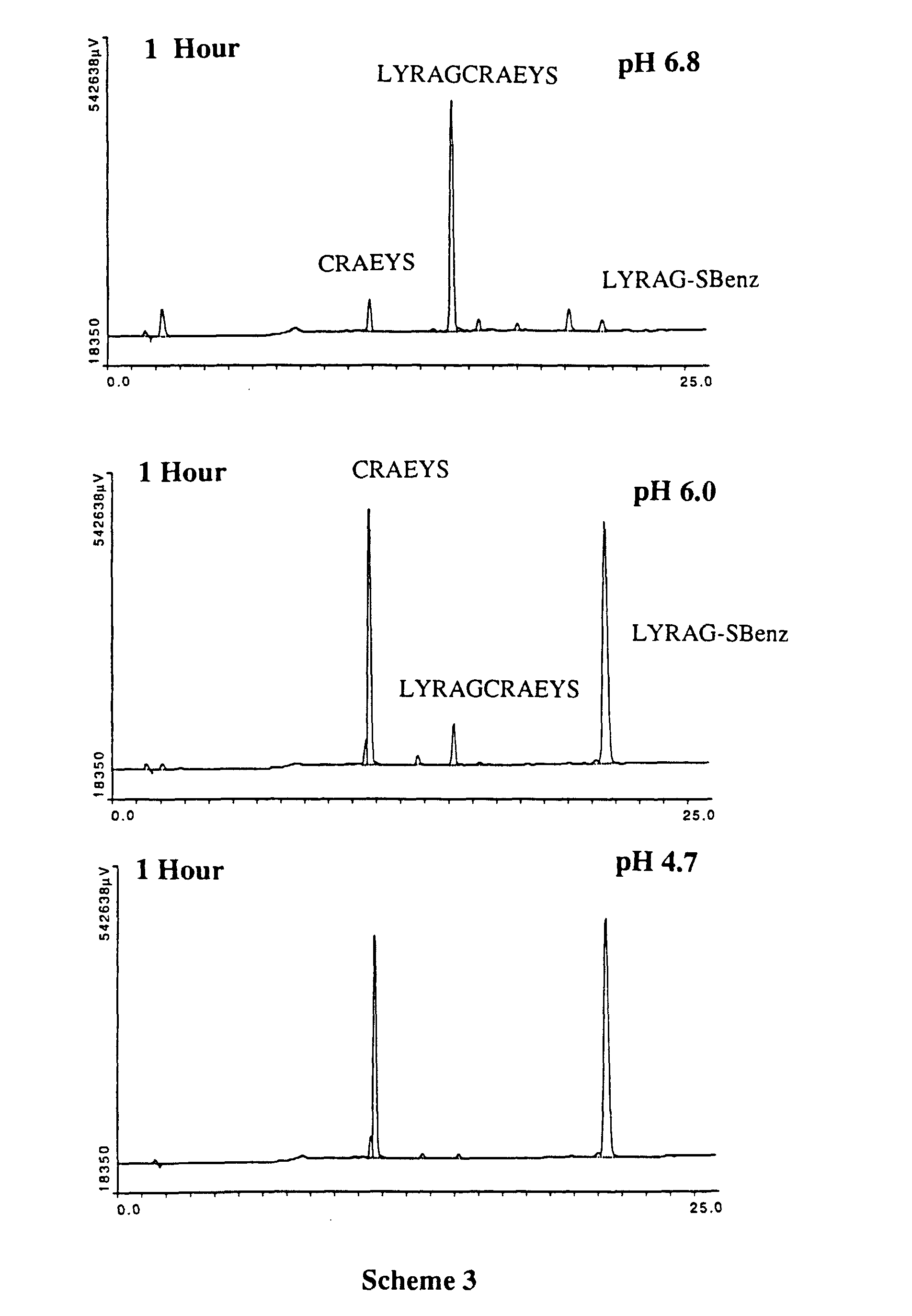 acetate buffer. The benzyl thioester is then purified by RPHPLC.

The conditions stated above, permit the formation of an unprotected oligonucleotide which is equipped with the activated thioester. Subsequent reaction with a second peptide containing a terminal cysteine residue, permits a facile coupling with the formation of a native peptide bond and can generate oligopeptide chains of 100 or more amino acid residues (Scheme 1).

### Examples

### Example 1

The model peptide Leu-Tyr-Arg-Ala-Gly-αCOSH (Sequence No.: 1) is prepared by optimized stepwise solid-phase peptide synthesis on an aminomethyl resin. The thioester resin linker is prepared by a generalized version as adopted from Kent et. al. *Tetrahedron Letters,* (1995): vol. 36, p 1217; J. Blake, *Int. J. Pept. Protein Res.* (1981): vol. 17, p 273; D. Yamashiro and C.H. Li, *ibid.* (1988): vol. 31, p 322. Once the desired chain is synthesized, the peptide resin (approx. 45 µmol original load) is treated in 8 mL liquid HF (0.8 mL anisole) at 0 °C for 1 hour. After evaporation with nitrogen, the residue is washed with ethyl acetate. The solid is subsequently stirred in water (approx. 15 mL) at 0 °C while adjusting the pH to 6.0 with solid ammonium bicarbonate. Filtration and lyophilization gives the crude thioacid product which can be further purified by preparative HPLC in 30 mg batches.

The thioester terminal peptide segment is subsequently prepared from the thioacid fragment by chemical synthesis to equip it with the necessary α-COSR functionality where R is an alkyl group such as benzyl, 5-thio-2-nitrobenzoic acid (-SNB), thiophenol, etc. The use of better thioester leaving groups resulted in faster ligation reactions. Thus, the model peptide Leu-Tyr-Arg-Ala-Gly-αCOSH (Sequence No.: 1) is first converted to the thiobenzylester by reaction with benzyl bromide (15 equivalents) in 6.0 M guanidine-HCl, pH 4.6, sodium acetate buffer to form Leu-Tyr-Arg-Ala-Gly-αCOSBn (Sequence No.: 3). The resulting peptide is purified under standard reversed-phase high-performance liquid chromatography (HPLC) conditions using approximately 20-45% acetonitrile at 1% per minute; monitored at 214 nm.

For the peptide H-Cys-Arg-Ala-Glu-Tyr-Ser (Sequence No.: 2), solid phase methods allow the preparation of peptides of up to 60 residues in good yield and high purity as described in M. Schnolzer, P. Alewood, D. Alewood, S.B. H. Kent, *Int. J. Pept. Protein Res.* (1992): vol. 40, 180.

First, to explore the mechanism of the reaction, the peptide Leu-Tyr-Arg-Ala-Gly-αCOSBn (Bn, benzyl; sequence No.: 3) was reacted with Ac-Cys (containing a blocked α-NH₂ functional group - commercially available from Novabiochem corp.). The exact mass of the resulting ligation product, Leu-Tyr-Arg-Ala-Gly-αCOS-CH₂C(NHAc)CO₂H (Sequence No.: 4), was determined by electrospray mass spectrometry and was consistent with a thioester-linked peptide as the ligation product generated by nucleophilic attack of the Ac-Cys side chain on the α-thioester moiety of the peptide.

Finally, the reaction of Leu-Tyr-Arg-Ala-Gly-αCOSBn (Sequence No.: 3) with Cys-Arg-Ala-Glu-Tyr-Ser (Sequence No.: 2, containing an unblocked α-NH₂ functional group) proceeded rapidly at pH 6.8 (below pH 6.0, the reaction proceeded very slowly, suggesting the involvement of the ionized thiolate of the Cys side chain at pH 6.8; scheme 3) and gave a single product of the expected mass. The peptides Leu-Tyr-Arg-Ala-Gly-αCOSBn + Cys-Arg-Ala-Glu-Tyr-Ser were reacted in 0.1 M phosphate buffer at pH 6.8, 6.0 and 4.7 at 25 °C. After 1 hour, the reactions had proceeded as follows: at pH 6.8 >95%; at pH 6.0 approximately 10%; and at pH 4.7, approximately 1.0% of ligated product Leu-Tyr-Arg-Ala-Gly-Cys-Arg-Ala-Glu-Tyr-Ser (Sequence No.: 5). As observed by HPLC, scheme 3 shows the pH dependence of the reaction after 1 hour and at 25 °C. This product lacked susceptibility to nucleophiles and had the ability to form disulfide-linked dimeric peptides, indicating unambiguously the formation of a native amide bond at the ligation site.

Another, unpublished model using the 2-thioacetic acid derivative Leu-Tyr-Arg-Ala-Gly-SCH₂COOH (Sequence No.: 6 - ,formed from attack of the thioacid Leu-Tyr-Arg-Ala-Gly-SH (Sequence No.: 1), onto 2-bromoacetic acid in methylene chloride) + Cys-Arg-Ala-Glu-Tyr-Ser (Sequence No.: 2) was ligated at pH 6.8 in 0.2 M phosphate buffer, at 45 °C. After 1.0 hour the reaction had proceeded to 80% as observed in scheme 4 by HPLC. The isolation of oxidation products from the ligated Leu-Tyr-Arg-Ala-Gly-Cys-Arg-Ala-Glu-Tyr-Ser (Sequence No.: 5) and unreacted Cys-Arg-Ala-Glu-Tyr-Ser demonstrated the presence of a free thiol ligation product.

The native chemical ligation procedure is generally applicable to peptides containing the full range of functional groups, normally found in proteins. Even free internal Cys residues may be present in either of the reacting segments. Internal Cys residues can undergo ester exchange with the peptide-α-thioester component; however, this reaction is unproductive because no rearrangement to the amide bond can occur, the thioester formed is readily reversible and remains a productive part of the reacting system.

The native chemical ligation procedure is limited to reaction at an amino-terminal Cys residue. To prevent the side chain thiol of this Cys from oxidizing to form a disulfide-linked dimer, an excess of thiol corresponding to the thioester leaving group is used to keep the Cys residues in reduced form without interfering with the ligation reaction. In addition, small amounts of low molecular weight thiols such as thiophenol are added to the coupling reaction mixture to maintain a reducing environment.

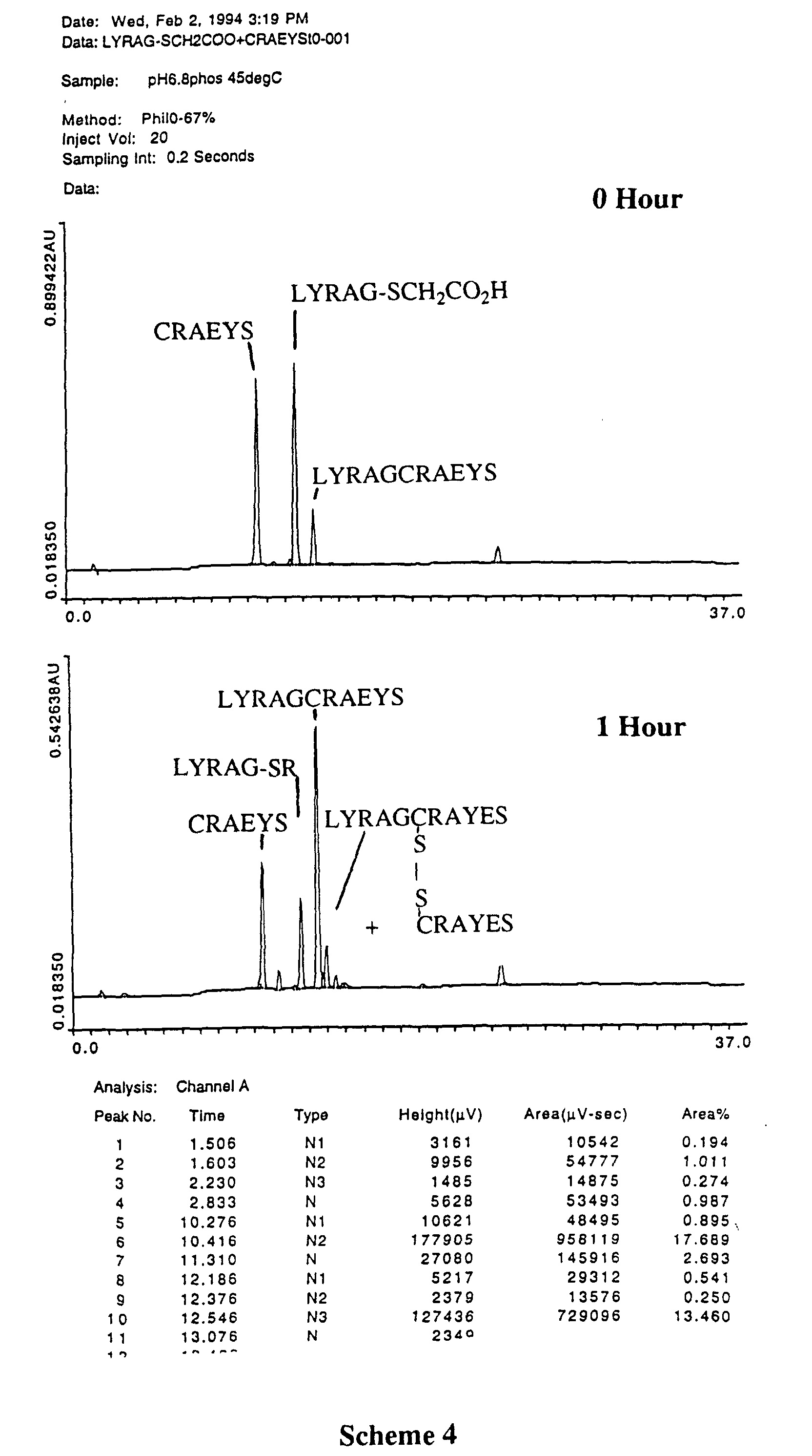

The addition of thiols increases the reactivity of the thioester, particularly if the added thiol is a better leaving group than the pre-formed thioester. An example of this observation is when the benzyl ester is converted to a phenyl ester by addition of thiophenol to reaction. Reaction yields and rates are substantially increased. For example, after 7 hours with benzyl mercaptan, the Barnase reaction yielded 25%, while the thiophenol treatment to the same reaction mixture yielded 90%).

Addition of thiols to the ligation mixture also keeps the reaction mixture in a reduced form. This prevents oxidation of the reactive N-terminal Cys residues and when internal Cys residues are present, the thiols reduce the formation of intramolecular disulfide bonds. Additionally, the reducing environment increases the stability of the thioester segment (ligation reactions can proceed overnight with little or no hydrolysis at pH 7.5).

### Example 2: HIV-1 K41 protease (unpublished conditions)

Ligation reactions are performed in several ways. An optimized procedure for a ligation reaction involving a (5-thio-2-nitrobenzoic acid) SNB thioester is to weigh the two peptides, HIV (1-40)-COSNB (Sequence No.: 11, formed from standard conditions stated herein) and HIV (41-99) (Sequence No.: 12, formed from standard conditions stated herein), as solids in the same reaction vessel and add 6.0 M guanidine HCl pH 6.5 with 100 mM Na acetate (the approximate peptide concentration is 7-13 mg/mL of each peptide).

After 5 min, approx. 2.0 % thiol is added. Two thiol catalysts have been used, viz. benzyl mercaptan (forms the benzyl thioester insitu; Sequence No.: 13) and thiophenol (forms the phenyl thioester insitu; Sequence No.: 14). In the ligation of HIV PR, reaction with benzyl mercaptan gave greater than 60% product yield in 40 hours while the thiophenol gave greater than 80% product yield in 10 hours to form HIV-1 K41 protease (Sequence No.: 15).

Subsequent treatment with TCEP was found to aid in the purification and analysis of product by reducing the thiophenol and benzyl mercaptan disulfide products which tend to co-elute with peptide products. The product was purified by standard reversed-phase HPLC as described via *supra* and characterized by electrospray mass spectroscopy (Scheme 9).

### Example 3. Barnase example (unpublished conditions)

The two peptides Barnase(1-48)-SNB (Sequence No.: 16, formed from standard conditions stated herein) and Barnase(49-110), (Sequence No.: 17, formed from standard conditions stated herein) were weighed as solids in the same reaction vessel and dissolved in pH 7.5 buffer (6M Guanidine 100mM phosphate). Immediately upon dissolving the peptides, 2% benzyl mercaptan (forms the benzyl thioester insitu; Sequence No.: 18) or 4% thiophenol (forms the phenyl thioester insitu; Sequence No.: 19) was added. After 7 hours, the benzyl mercaptan reaction proceeded 25% and the thiophenol reaction proceeded to > 90% to form Barnase (1-110) (Sequence No.: 20). The product was purified by standard reversed-phase HPLC as described via supra (scheme 10).

The addition of thiols increases the reactivity of the thioester, particularly if the added thiol is a better leaving group than the pre-formed thioester. An example of this observation is when the benzyl ester is converted to a phenyl ester by addition of thiophenol to reaction. Reaction yields and rates are substantially increased. For example, after 7 hours with benzyl mercaptan, the Barnase reaction yielded 25%, while the thiophenol treatment to the same reaction mixture yielded 90% to form Barnase (1-110) (Sequence No.: 20).

Addition of thiols to the ligation mixture also keeps the reaction mixture in a reduced form. This prevents oxidation of the reactive N-terminal Cys residues and 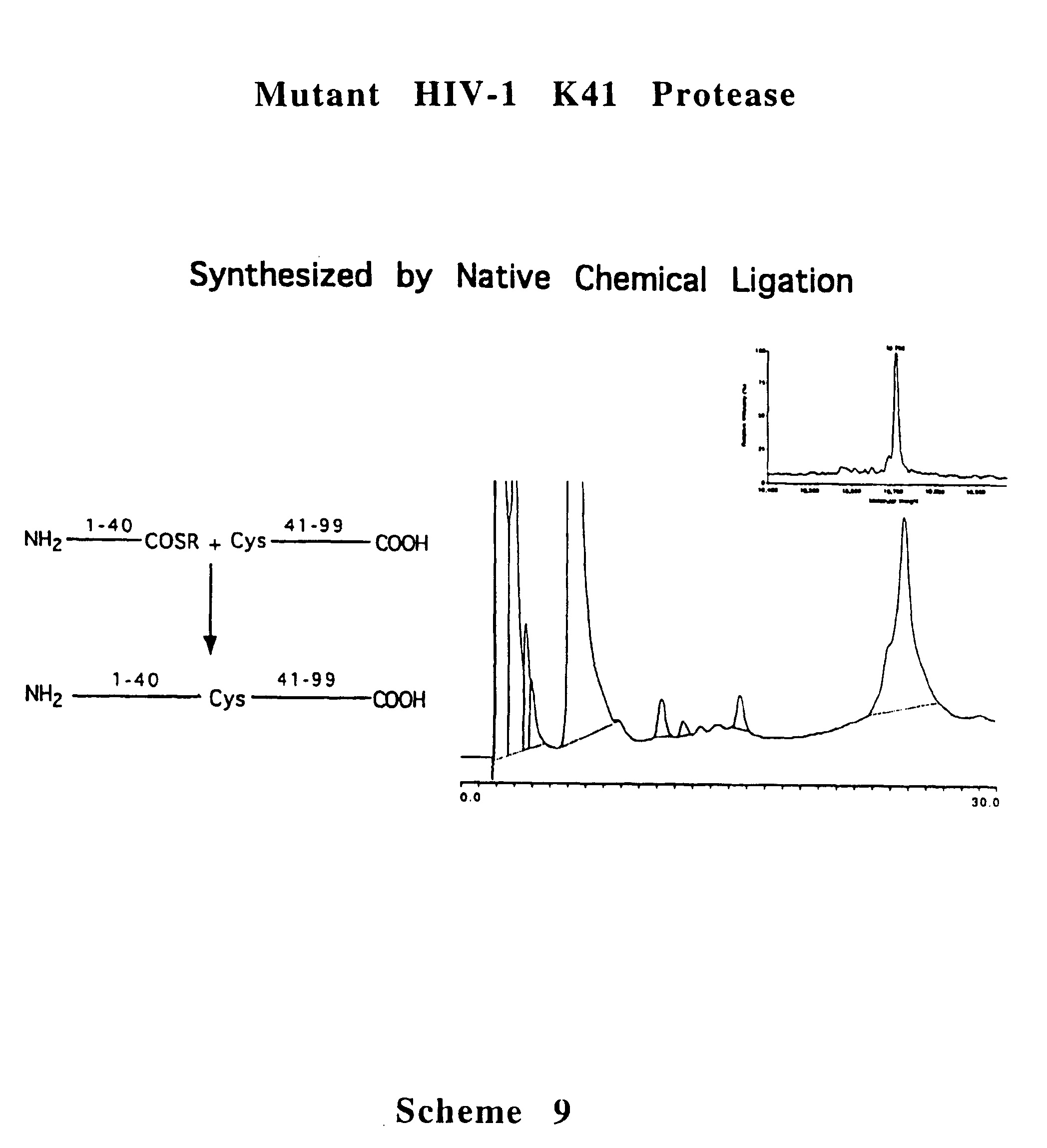 when internal Cys residues are present, the thiols reduce the formation of intramolecular disulfide bonds. Additionally, the reducing environment increases the stability of the thioester segment (ligation reactions can proceed overnight with little or no hydrolysis at pH 7.5).

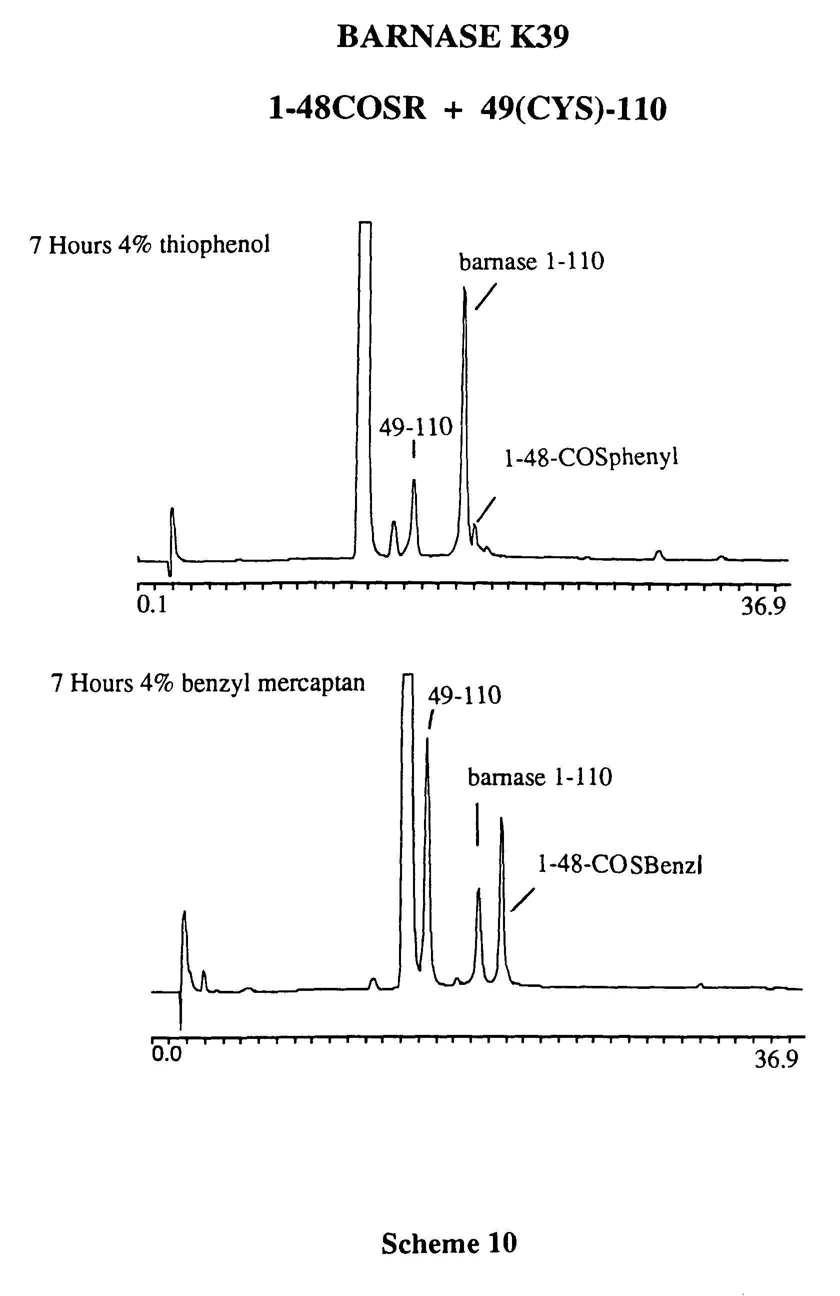

## Claims

1. A method for ligating a first oligopeptide with a second oligopeptide end to end for producing an oligopeptide product, the method comprising the following steps:
Step A: admixing the first and second oligopeptide in a reaction solution, the first oligopeptide including a C-terminal thioester, the second oligopeptide including an N-terminal cysteine having an unoxidized sulfhydryl side chain; then
Step B: condensing the unoxidized sulfhydryl side chain of the N-terminal cysteine with the C-terminal thioester for producing an intermediate oligopeptide linking the first and second oligopeptide with a β-aminothioester bond; and then
Step C: rearranging the β-aminothioester bond of the intermediate oligopeptide peptide of said Step B for producing the oligopeptide product linking the first and second oligopeptides with an amide bond,
characterised in that a catalytic thiol is added to the reaction solution in Step A, the catalytic thiol (a) being a conjugated thiol or thiolate attached directly to an aromatic or hetero-aromatic ring, (b) being present in a catalytic concentration and (c) being different to the thiol from which the said C-terminal thioester is notionally formed.

2. The method as described in claim 1 wherein the catalytic thiol is a better leaving group than the thiol from which the C-terminal thioester is notionally formed.

3. The method as described in Claim 1 or claim 2 wherein, in said step A, the catalytic thiol is selected from the group consisting of thiophenol, 1-thio-2-nitrophenol, 2-thio-benzoic acid, 2-thio-pyridine, 4-thio-2-pyridinecarboxylic acid, and 4-thio-2-nitro-pyridine.

4. The method as described in Claim 3 wherein, in said step A, the conjugated thiol is thiophenol.

5. A method of any one of claims 1 to 4, wherein the oligopeptide having a C-terminal thioester is produced by a method comprising the following steps:
Step A: providing a resin having a linker with an unoxidized thiol;
Step B: providing a Boc-amino acid succinimide ester; then
Step C: admixing the resin of said Step A and the Boc-amino acid succinimide ester of said Step B under reaction conditions for producing a Boc-amino thioester-resin; then
Step D: assembling an oligopeptide onto the Boc-amino thioester-resin by stepwise solid phase peptide synthesis; then
Step E: cleaving the Boc-amino thioester-resin of said Step D with HF for producing an oligopeptide having a C-terminal thiol; and then
Step F: converting the oligopeptide having a C-terminal thiol of said Step E to the oligopeptide having a C-terminal thioester.

## Patentansprüche

1. Verfahren zum Ligieren eines ersten Oligopeptids mit einem zweiten Oligopeptid Ende an Ende zur Herstellung eine Oligopeptid-Produkts, wobei das Verfahren die folgenden Schritte umfasst:
Schritt A: Mischen des ersten und zweiten Oligopeptids in einer Reaktionslösung, wobei das erste Oligopeptid einen C-terminalen Thioester einschliesst, das zweite Oligopeptid ein N-terminales Cystein mit einer nichtoxidierten Sulfhydryl-Seitenkette einschliesst; danach
Schritt B: Kondensieren der nichtoxidierten Sulfhydryl-Seitenkette des N-terminalen Cysteins mit dem C-terminalen Thioester zur Bildung eines intermediären Oligopeptids, welches das erste und zweite Oligopeptid mit einer β-Aminothioester-Bindung verbindet; und im Anschluss
Schritt C: Umgruppieren der β-Aminothioester-Bindung des intermediären Oligopeptid-Peptids von Schritt B zur Herstellung des Oligopeptid-Produkts, welches das erste und das zweite Oligopeptide mit einer Amidbindung verbindet,
dadurch gekennzeichnet, dass ein katalytisches Thiol der Reaktionslösung in Schritt A hinzugegeben wird, wobei das katalytische Thiol (a) ein konjugiertes Thiol oder Thiolat ist, welches direkt an einem aromatischen oder heteroaromatischen Ring gebunden ist, (b) in einer katalytischen Konzentration vorliegt, und (c) sich von dem Thiol unterscheidet, aus welchem der C-terminale Thioester rein gedanklich gebildet ist.

2. Verfahren wie in Anspruch 1 beschrieben, in welchem das katalytische Thiol eine bessere Abgangsgruppe ist als das Thiol, aus welchem der C-terminale Thioester rein gedanklich gebildet ist.

3. Verfahren wie in Anspruch 1 oder Anspruch 2 beschrieben, in welchem in Schritt A das katalytische Thiol gewählt ist aus der Thiophenol, 1-Thio-2-nitrophenol, 2-Thiobenzoesäure, 2-Thiopyridin, 4-Thio-2-pyridincarbonsäure und 4-Thio-2-nitropyridin umfassenden Gruppe.

4. Verfahren wie in Anspruch 3 beschrieben, in welchem in Schritt A das konjugierte Thiol Thiophenol ist.

5. Verfahren nach mindestens einem der Ansprüche 1 bis 4, in welchem das Oligopeptid mit einem C-terminalen Thioester durch ein Verfahren hergestellt wird, umfassend die folgenden Schritte:
Schritt A: Vorsehen eines Harzes mit einem Linker mit einem nichtoxidierten Thiol;
Schritt B: Vorsehen eines Boc-Aminosäuresuccinimidesters; danach
Schritt C: Vermischen des Harzes aus Schritt A und des Boc-Aminosäuresuccinimidesters aus Schritt B unter Reaktionsbedingungen zur Herstellung eines Boc-Aminothioesterharzes; im Anschluss
Schritt D: Anbringen eines Oligopeptids auf dem Boc-Aminothioesterharz durch schrittweise Festphasen-Peptidsynthese; danach
Schritt E: Abspalten des Boc-Aminothioesterharzes von Schritt D mit HF zur Herstellung eines Oligopeptids mit einem C-terminalen Thiol; und danach
Schritt F: Umwandeln des Oligopeptids mit einem C-terminalen Thiol aus Schritt E zu dem Oligopeptid mit einem C-terminalen Thioester.

## Revendications

1. Procédé pour ligaturer bout à bout un premier oligopeptide avec un second oligopeptide pour produire un produit oligopeptide, ledit procédé étant constitué des étapes suivantes :
Etape A : mélanger les premier et second oligopeptides dans une solution de réaction, le premier oligopeptide comprenant un thioester C-terminal, le second oligopeptide comprenant une cystéine N-terminale ayant une chaîne latérale sulfhydryle non-oxydée ; puis
Etape B : condenser la chaîne latérale sulfhydryle non-oxydée de la cystéine N-terminale avec le thioester C-terminal de manière à produire un oligopeptide intermédiaire liant les premier et second oligopeptides par une liaison β-aminothioester ; puis
Etape C : réarranger la liaison β-aminothioester de l'oligopeptide intermédiaire obtenu à l'étape B de manière à produire le produit oligopeptide liant les premier et second oligopeptides par une liaison amide,
caractérisé en ce qu'un thiol catalytique est ajouté à la solution de réaction au cours de l'étape A, ledit thiol catalytique (a) étant un thiol ou un thiolate conjugué, directement lié à un cycle aromatique ou hétéro-aromatique, (b) étant présent en une concentration catalytique et (c) étant différent du thiol à partir duquel ledit thioester C-terminal est hypothétiquement formé.

2. Procédé selon la revendication 1, dans lequel le thiol catalytique est un meilleur groupement labile que le thiol à partir duquel le thioester C-terminal est hypothétiquement formé.

3. Procédé selon la revendication 1 ou 2 dans lequel, dans ladite étape A, le thiol catalytique est sélectionné dans le groupe consistant en le thiophénol, le 1-thio-2-nitrophénol, l'acide 2-thio-benzoïque, la 2-thio-pyridine, l'acide 4-thio-2-pyridine carboxylique, et la 4-thio-2-nitro-pyridine.

4. Procédé selon la revendication 3 dans lequel, dans ladite étape A, le thiol conjugué est un thiophénol.

5. Procédé selon l'une quelconque des revendications 1 à 4, dans lequel l'oligopeptide ayant un thioester C-terminal est produit par un procédé constitué des étapes suivantes :
Etape A : fournir une résine ayant un liant avec un thiol non-oxydé ;
Etape B : fournir un ester de succinimide d'un BOC-amino-acide ; puis
Etape C : mélanger la résine de ladite étape A et l'ester de succinimide du BOC-amino-acide de ladite étape B sous des conditions de réaction convenant à produire une résine BOC-amino-thioester ; puis
Etape D : assembler un oligopeptide à la résine BOC-amino-thioester par synthèse de peptides, par étapes, en phase solide ; puis
Etape E : cliver la résine BOC-amino-thioester de ladite étape D avec HF de manière à produire un oligopeptide ayant un thiol C-terminal ; puis
Etape F : convertir l'oligopeptide ayant un thiol C-terminal de ladite étape E en l'oligopeptide ayant un thioester C-terminal.


### SEQUENCE LISTING

(1) GENERAL INFORMATION:
   (i) APPLICANT:
      (A) NAME: The Scripps Research Institute
      (B) STREET: 10666 North Torrey Pines Road, Suite 220, Mail Drop TPC8
      (C) CITY: La Jolla
      (D) STATE: CA
      (E) COUNTRY: USA
      (F) POSTAL CODE (ZIP): 92037
      (G) TELEPHONE: 619-554-2937
      (H) TELEFAX: 619-554-6312
   (ii) TITLE OF INVENTION: SYNTHESIS OF PROTEINS BY NATIVE CHEMICAL LIGATION
   (iii) NUMBER OF SEQUENCES: 20
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentln Release #1.0, Version #1.25 (EPO)
   (v) CURRENT APPLICATION DATA:
      (A) APPLICATION NUMBER: PCT/US
      (B) FILING DATE: 04-MAY-1995
(2) INFORMATION FOR SEQ ID NO:1:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 5
      (D) OTHER INFORMATION: /label= COSH
         /note = "Wherein COSH is thioacid."
   (xii) SEQUENCE DESCRIPTION: SEQ ID NO:1:
   Leu Tyr Arg Ala Gly
   1               5
(2) INFORMATION FOR SEQ ID NO:2:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Cys Arg Ala Glu Tyr Ser
   1 5
(2) INFORMATION FOR SEQ ID NO:3:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 5
      (D) OTHER INFORMATION: /label= COSBn
         /note = "Wherein COSBn is benzyl thioester."
   (xi) SEQUENCE DESCRIPTION: SEQ ID NO:3:
   Leu Tyr Arg Ala Gly
   1 5
(2) INFORMATION FOR SEQ ID NO:4:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 5
      (D) OTHER INFORMATION: /label= X
         /note = "Wherein X is N-acetyl-cysteine-thioester."
   (xi) SEQUENCE DESCRIPTION: SEQ ID NO:4:
   Leu Tyr Arg Ala Gly
   1 5
(2) INFORMATION FOR SEQ ID NO:5:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:5:
   Leu Tyr Arg Ala Gly Cys Arg Ala Glu Tyr Ser
   1 5 10
(2) INFORMATION FOR SEQ ID NO:6:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 5
      (D) OTHER INFORMATION: /label= SCH2COOH
         /note = "Wherein SCH2COOH is 2-thioacetic acid."
   (xi) SEQUENCE DESCRIPTION: SEQ ID NO:6:
   Leu Tyr Arg Ala Gly
   1 5
(2) INFORMATION FOR SEQ ID NO:7:
   (i) SEQUENCE CHARACTERISTICS:
      (A) LENGTH: 33 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 33
      (D) OTHER INFORMATION: /label= COSH
         /note = "Wherein COSH is thioacid."
   (ix) FEATURE:
      (A) NAME/KEY: Modified-site
      (B) LOCATION: 1
      (D) OTHER INFORMATION: /label= Msc
         /note = "Wherein Msc is
         2-methyl-sulfonyl-ethyloxy-carbonyl."
   (xi) SEQUENCE DESCRIPTION: SEQ ID NO:7:
   Ser Ala Lys Glu Leu Arg Cys Gln Cys Ile Lys Thr Tyr Ser Lys Pro
   1 5 10 15
   Phe His Pro Lys Phe Ile Lys Glu Leu Arg Val Ile Glu Ser Gly Pro
   20 25 30
   Ala
(2) INFORMATION FOR SEQ ID NO:8:
   (i) SEQUENCE CHARACTERISTICS:
      (A) LENGTH: 33 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 33
      (D) OTHER INFORMATION: /label= COSBn
         /note = "Wherein COSBn is benzyl thioester."
   (xi) SEQUENCE DESCRIPTION: SEQ ID NO:8:
   Ser Ala Lys Glu Leu Arg Cys Gln Cys Ile Lys Thr Tyr Ser Lys Pro
   1 5 10 15
   Phe His Pro Lys Phe Ile Lys Glu Leu Arg Val Ile Glu Ser Gly Pro
   20 25 30
   Ala
(2) INFORMATION FOR SEQ ID NO:9:
   (i) SEQUENCE CHARACTERISTICS:
      (A) LENGTH: 39 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:9:
   Cys Ala Asn Thr Glu Ile Ile Val Lys Leu Ser Asp Gly Arg Glu Leu
   1 5 10 15
   Cys Leu Asp Pro Lys Glu Asn Trp Val Gln Arg Val Val Glu Lys Phe
   20 25 30
   Leu Lys Arg Ala Glu Asn Ser
   35
(2) INFORMATION FOR SEQ ID NO:10:
   (i) SEQUENCE CHARACTERISTICS:
      (A) LENGTH: 72 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 72
      (D) OTHER INFORMATION: /label= SH4
   (xi) SEQUENCE DESCRIPTION: SEQ ID NO:10:
   Ser Ala Lys Glu Leu Arg Cys Gln Cys Ile Lys Thr Tyr Ser Lys Pro
   1 5 10 15
   Phe His Pro Lys Phe Ile Lys Glu Leu Arg Val Ile Glu Ser Gly Pro
   20 25 30
   Ala Cys Ala Asn Thr Glu Ile Ile Val Lys Leu Ser Asp Gly Arg Glu
   35 40 45
   Leu Cys Leu Asp Pro Lys Glu Asn Trp Val Gln Arg Val Val Glu Lys
   50 55 60
   Phe Leu Lys Arg Ala Glu Asn Ser
   65 70
(2) INFORMATION FOR SEQ ID NO:11:
   (i) SEQUENCE CHARACTERISTICS:
      (A) LENGTH: 40 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 40
      (D) OTHER INFORMATION: /label= COSNB
         /note = "Wherein COSNB is 5-thio-2-nitro-benzoic
         acid ester."
   (ix) FEATURE:
      (A) NAME/KEY: Modified-site
      (B) LOCATION: 27
      (D) OTHER INFORMATION: /label= Xaa
         /note = "Wherein Xaa is 2-Aminobutyric acid."
   (xi) SEQUENCE DESCRIPTION: SEQ ID NO:11:
   Pro Gln Ile Thr Leu Trp Lys Arg Pro Leu Val Thr Ile Arg Ile Gly
   1 5 10 15
   Gly Gln Leu Lys Glu Ala Leu Leu Asp Thr Gly Ala Asp Asp Thr Val
   20 25 30
   Ile Glu Glu Met Asn Leu Pro Gly
   35 40
(2) INFORMATION FOR SEQ ID NO:12:
   (i) SEQUENCE CHARACTERISTICS:
      (A) LENGTH: 59 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 27
      (D) OTHER INFORMATION: /label= Xaa
         /note = "Wherein Xaa is 2-Aminobutyric acid."
   (ix) FEATURE:
      (A) NAME/KEY: Modified-site
      (B) LOCATION: 55
      (D) OTHER INFORMATION: /label= Xaa
         /note = "Wherein Xaa is 2-Aminobutyric acid."
   (xi) SEQUENCE DESCRIPTION: SEQ ID NO:12:
   Cys Trp Lys Pro Lys Met Ile Gly Gly Ile Gly Gly Phe Ile Lys Val
   1 5 10 15
   Arg Gln Tyr Asp Gln Ile Pro Val Glu Ile Xaa Gly His Lys Ala Ile
   20 25 30
   Gly Thr Val Leu Val Gly Pro Thr Pro Val Asn Ile Ile Gly Arg Asn
   35 40 45
   Leu Leu Thr Gln Ile Gly Xaa Thr Leu Asn Phe
   50 55
(2) INFORMATION FOR SEQ ID NO:13:
   (i) SEQUENCE CHARACTERISTICS:
      (A) LENGTH: 40 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 40
      (D) OTHER INFORMATION: /label= COSBn
         /note= "Wherein COSBn is ??."
   (ix) FEATURE:
      (A) NAME/KEY: Modified-site
      (B) LOCATION: 40
      (D) OTHER INFORMATION: /label= COSBn
         /note = "Wherein COSBn is benzyl thio ester."
   (xi) SEQUENCE DESCRIPTION: SEQ ID NO:13:
   Pro Gln Ile Thr Leu Trp Lys Arg Pro Leu Val Thr Ile Arg Ile Gly
   1               5                   10                  15
   Gly Gln Leu Lys Glu Ala Leu Leu Asp Thr Gly Ala Asp Asp Thr Val
               20                  25                  30
   Ile Glu Glu Met Asn Leu Pro Gly
           35                  40
(2) INFORMATION FOR SEQ ID NO:14:
   (i) SEQUENCE CHARACTERISTICS:
      (A) LENGTH: 40 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 40
      (D) OTHER INFORMATION: /label= COSPh
         /note = "Wherein COSPh is phenyl thioester."
   (xi) SEQUENCE DESCRIPTION: SEQ ID NO:14:
(2) INFORMATION FOR SEQ ID NO: 15:
   (i) SEQUENCE CHARACTERISTICS:
      (A) LENGTH: 99 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 67
      (D) OTHER INFORMATION: /label= Xaa
         /note = "Wherein Xaa is amino butyric acid."
   (ix) FEATURE:
      (A) NAME/KEY: Modified-site
      (B) LOCATION: 95
      (D) OTHER INFORMATION: /label= Xaa
         /note = "Wherein Xaa is 2-Aminobutyric acid."
   (xi) SEQUENCE DESCRIPTION: SEQ ID NO:15:
   Pro Gln Ile Thr Leu Trp Lys Arg Pro Leu Val Thr Ile Arg Ile Gly
   1 5 10 15
   Gly Gln Leu Lys Glu Ala Leu Leu Asp Thr Gly Ala Asp Asp Thr Val
   20 25 30
   Ile Glu Glu Met Asn Leu Pro Gly Cys Trp Lys Pro Lys Met Ile Gly
   35 40 45
   Gly Ile Gly Gly Phe Ile Lys Val Arg Gln Tyr Asp Gln Ile Pro Val
   50 55 60
   Glu Ile Xaa Gly His Lys Ala Ile Gly Thr Val Leu Val Gly Pro Thr
   65 70 75 80
   Pro Val Asn Ile Ile Gly Arg Asn Leu Leu Thr Gln Ile Gly Xaa Thr
   85 90 95
   Leu Asn Phe
(2) INFORMATION FOR SEQ ID NO:16:
   (i) SEQUENCE CHARACTERISTICS:
      (A) LENGTH: 48 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 48
      (D) OTHER INFORMATION: /label= COSNB
         /note = "Wherein COSNB is 5-thio-2-nitro benzoic acid ester."
   (xi) SEQUENCE DESCRIPTION: SED ID NO:16:
   Ala Gln Val Ile Asn Thr Phe Asp Gly Val Ala Asp Tyr Leu Gln Thr
   1 5 10 15
   Tyr His Lys Leu Pro Asn Asp Tyr Ile Thr Lys Ser Glu Ala Gln Ala
   20 25 30
   Leu Gly Trp Val Ala Ser Lys Gly Asn Leu Ala Asp Val Ala Pro Gly
   35 40 45
(2) INFORMATION FOR SEQ ID NO:17:
   (i) SEQUENCE CHARACTERISTICS:
      (A) LENGTH: 62 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SED ID NO:17:
   Cys Ser Ile Gly Gly Asp Ile Phe Ser Asn Arg Glu Gly Lys Leu Pro
   1 5 10 15
   Gly Lys Ser Gly Arg Thr Trp Arg Glu Ala Asp Ile Asn Tyr Thr Ser
   20 25 30
   Gly Phe Arg Asn Ser Asp Arg Ile Leu Tyr Ser Ser Asp Trp Leu Ile
   35 40 45
   Tyr Lys Thr Thr Asp His Tyr Gln Thr Phe Thr Lys Ile Arg
   50 55 60
(2) INFORMATION FOR SEQ ID NO:18:
   (i) SEQUENCE CHARACTERISTICS:
      (A) LENGTH: 48 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 48
      (D) OTHER INFORMATION: /label= COSBn
         /note = "Wherein COSBn is benzyl thio ester."
   (xi) SEQUENCE DESCRIPTION: SED ID NO:18:
   Ala Gln Val Ile Asn Thr Phe Asp Gly Val Ala Asp Tyr Leu Gln Thr
   1 5 10 15
   Tyr His Lys Leu Pro Asn Asp Tyr Ile Thr Lys Ser Glu Ala Gln Ala
   20 25 30
   Leu Gly Trp Val Ala Ser Lys Gly Asn Leu Ala Asp Val Ala Pro Gly
   35 40 45
(2) INFORMATION FOR SEQ ID NO:19:
   (i) SEQUENCE CHARACTERISTICS:
      (A) LENGTH: 48 amino acids
      (B) TYPE: amino acid
      (C) STRANDEDNESS: single
      (D) TOPOLOGY: linear
   (ii) MOLECULE TYPE: peptide
   (ix) FEATURE:
      (A) NAME/KEY: Modified-site
      (B) LOCATION: 48
      (D) OTHER INFORMATION: /label= COSPh
         /note = "Wherein COSPh is phenyl thio ester."
   (xi) SEQUENCE DESCRIPTION: SED ID NO:19:
   Ala Gln Val Ile Asn Thr Phe Asp Gly Val Ala Asp Tyr Leu Gln Thr
   1 5 10 15
   Tyr His Lys Leu Pro Asn Asp Tyr Ile Thr Lys Ser Glu Ala Gln Ala
   20 25 30
   Leu Gly Trp Val Ala Ser Lys Gly Asn Leu Ala Asp Val Ala Pro Gly
   35 40 45
(2) INFORMATION FOR SEQ ID NO:20:
   (i) SEQUENCE CHARACTERISTICS:
      (A) LENGTH: 110 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SED ID NO:20:
   Ala Gln Val Ile Asn Thr Phe Asp Gly Val Ala Asp Tyr Leu Gln Thr
   1 5 10 15
   Tyr His Lys Leu Pro Asn Asp Tyr Ile Thr Lys Ser Glu Ala Gln Ala
   20 25 30
   Leu Gly Trp Val Ala Ser Lys Gly Asn Leu Ala Asp Val Ala Pro Gly
   35 40 45
   Cys Ser Ile Gly Gly Asp Ile Phe Ser Asn Arg Glu Gly Lys Leu Pro
   50 55 60
   Gly Lys Ser Gly Arg Thr Trp Arg Glu Ala Asp Ile Asn Tyr Thr Ser
   65 70 75 80
   Gly Phe Arg Asn Ser Asp Arg Ile Leu Tyr Ser Ser Asp Trp Leu Ile
   85 90 95
   Tyr Lys Thr Thr Asp His Tyr Gln Thr Phe Thr Lys Ile Arg
   100 105 110